(19) Europäisches Patentamt
European Patent Office
Office européen des brevets

(11) Veröffentlichungsnummer : **0 044 009**
**B1**

(12) **EUROPÄISCHE PATENTSCHRIFT**

(45) Veröffentlichungstag der Patentschrift : **26.09.84**

(21) Anmeldenummer : **81105204.2**

(22) Anmeldetag : **04.07.81**

(51) Int. Cl.³ : **C 07 D249/06**, C 07 D249/08, C 07 D231/12// C07D249/22, C07D249/20

(54) **Verfahren zur Herstellung von 2-(2,2,2-Trichlor-1-hydroxy-ethyl)-phenolen.**

(30) Priorität : **16.07.80 DE 3026958**

(43) Veröffentlichungstag der Anmeldung : **20.01.82 Patentblatt 82/03**

(45) Bekanntmachung des Hinweises auf die Patenterteilung : **26.09.84 Patentblatt 84/39**

(84) Benannte Vertragsstaaten : **CH DE FR GB IT LI**

(56) Entgegenhaltungen :
**DE-A- 2 255 290**
**DE-A- 2 335 218**
**DE-A- 2 848 670**
**HOUBEN WEYL: "Methoden der Organischen Chemie", Band VII, 1954, Teil 1, Georg Thieme Verlag Stuttgart, DE. O. BAYER: "Methoden zur Herstellung und Unwandlung von Aldehyden" Seiten 307-361**
**SYNTHESIS 824 (1979) ANGEWANDTE CHEMIE, BAND 90, 727 (1978)**

(73) Patentinhaber : **BAYER AG**
**Konzernverwaltung RP Patentabteilung**
**D-5090 Leverkusen 1 Bayerwerk (DE)**

(72) Erfinder : **Berneth, Horst, Dr.**
**Bergstrasse 47**
**D-5068 Odenthal (DE)**
Erfinder : **Raue, Roderich, Dr.**
**Berta-von-Suttner Strasse 48**
**D-5090 Leverkusen (DE)**

**EP 0 044 009 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung ist ein Verfahren zur Herstellung von 2-(2,2,2-Trichlor-1-hydroxy-ethyl)-phenolen der Formel

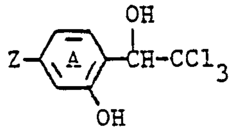

(I)

worin

Z einen Rest der 1,2-Diazol-1-yl-, 1,2,3-Triazol-1-yl-, 1,2,3-Triazol-2-yl-, 1,2,4-Triazol-1-yl- und 1,2,4-Triazol-4-yl-Reihe und

A einen Benzolring, der gegebenenfalls durch $C_1$-bis $C_2$-Alkyl, $C_1$- bis $C_2$-Alkoxy, Halogen oder einen anellierten Benzolring substituiert sein kann, bedeuten.

Das Verfahren ist dadurch gekennzeichnet, daß man Phenole Formel

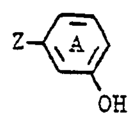

(IV)

worin

Z und A die oben angegebene Bedeutung besitzen, in Gegenwart von 0.02-0.1 Moläquivalenten ihrer Alkalialkalisalze und Lewis-Säuren mit Trichloracetaldehyd umsetzt.

Vorzugsweise führt man die Reaktion in einem unpolaren oder wenig polaren Lösungsmittel in Gegenwart von Lewis-Säuren der Formel

$$M'X_n \qquad (VI)$$

worin

M' ein Metall- oder Halbmetallatom der Gruppen IIb, IIIa, IVa, IVb oder VIII des periodischen Systems der Elemente,

X ein Halogenatom und

n eine ganze Zahl zwischen 2 und 4 bedeuten,

durch.

Unter unpolaren oder wenig polaren Lösungsmitteln sind solche zu verstehen, die üblicherweise bei Friedel-Crafts-Reaktionen stark aktivierter Aromaten Verwendung finden, nämlich geeignet substituierte Benzol-Derivate, mehrfach halogenierte $C_1$- bis $C_3$-Alkane und Schwefelkohlenstoff, beispielsweise Toluol, Chlorbenzol, 1,2-Dichlor-benzol, Nitrobenzol, Tetrachlorkohlenstoff und 1,2-Dichlor-ethan, vorzugsweise Toluol und Chlorbenzol.

Beispiele für Alkali- und Erdalkaliatome sind :

Lithium, Natrium, Kalium, Rubidium, Magnesium, Calcium, Strontium und Barium. Bevorzugt sind Natrium und Kalium.

Beispiele für Metall- und Halbmetallatome der Gruppen IIb, IIIa, IVa, IVb oder VIII des periodischen Systems der Elemente sind :

Zink, Bor, Aluminium, Gallium, Zinn, Titan, Eisen, Beispiele für Halogenatome sind Fluor, Chlor und Brom.

Bevorzugte Lewis-Säuren sind dann :

$ZnCl_2$, $BF_3$, $BCl_3$, $AlCl_3$, $AlBr_3$, $SnCl_4$, $TiCl_4$, $FeCl_3$.

Besonders bevorzugt ist $AlCl_3$.

Im einzelnen geht man zweckmäßigerweise so vor, daß man ein Phenol der Formel IV mit einer noch näher zu beschreibenden Menge von beispielsweise Natriumhydroxid- oder Kaliumhydroxid-Pulver in einem Lösungsmittel, wie z. B. Toluol, unter Auskreisung des Reaktionswassers erhitzt, die erhaltene Mischung aus Phenol und beispielsweise Natrium-bzw. kaliumphenolat in diesem Lösungsmittel mit einer noch näher zu beschreibenden Menge von z. B. Aluminiumtrichlorid vorzugsweise bei höherer Temperatur (30 °C bis Rückflußtemperatur des Lösungsmittels) zur Reaktion bringt und dann Trichloracetaldehyd zusetzt. Nach mehreren Stunden Reaktionszeit, wobei die Temperatur im allgemeinen unter 120 °C liegen sollte, isoliert man entweder direkt oder nach Behandeln mit wäßriger Ammoniumchloridlösung das entsprechende 2-(2,2,2-Trichlor-1-hydroxy-ethyl)-phenol der Formel I.

Durch die Verwendung der geringen Lewis-Säure-Mengen (z. B. $AlCl_3$) wird die direkte Isolierung der Produkte der Formel I aus der Reaktionslösung vorteilhaft.

Durch die Verwendung der geringen Alkalihydroxidmengen kann in hoch konzentrierter Lösung gearbeitet werden, da nur relativ geringe Mengen der sehr voluminösen Phenolate anfallen.

Überraschend ist, daß diese technisch bedeutsame katalytische Verwendung von Alkalihydroxid und Lewis-Säure bisher nicht erkannt wurde, sondern vielmehr gerade die Verwendung von jeweils 0,5 Moläquivalenten Kalium-Metall (zur Erzeugung des Phenolats) und Aluminiumtrichlorid als essentiell angegeben wurden (Synthesis *1979*, 824, vgl. auch Angew. Chem. *90*, 727 (1978)).

Bevorzugte Verfahrensprodukte sind solche der Formel

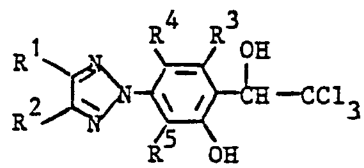

(II)

worin

$R^1$ und $R^2$ unabhängig voneinander Wasserstoff, einen gegebenenfalls substituierten Alkyl-, Cycloalkyl-, Aralkyl-, Hetaralkyl-, Aryl-, Hetaryl-, teilweise oder vollständig hydrierten Hetaryl-, Alkenyl-, Arylalkenyl- oder Hetarylalkenyl-Rest, Perhalogenalkyl, Hydroxy, einen gegebenenfalls substituierten Alkoxy- oder Alkylthio-Rest, Halogen, einen gegebenenfalls substituierten Amino-Rest, Cyano, einen gegebenenfalls substituierten Alkoxycarbonyl-, Aryloxycarbonyl-, Carbamyl-, Alkylsulfonyl-, Arylsulfonyl-, Hetarylsulfonyl-, Aminosulfonyl- oder Acyl-Rest, Dialkoxyphosphoryl oder gemeinsam die noch fehlenden Glieder eines gegebenenfalls substituierten oder Ketogruppen und/oder ein oder mehrere Heteroatome enthaltenden, gegebenenfalls durch einen gegebenenfalls substituierten Benzol-Ring oder Heterocyclus anellierten, ungesättigten oder teilweise oder vollständig gesättigten Ring und

$R^3$, $R^4$ und $R^5$ unabhängig voneinander Wasserstoff, Methyl, Methoxy oder Chlor bedeuten,

wobei die genannten Reste und Ringe vorzugsweise die nachstehend angegebenen spezielleren Bedeutungen haben.

Unter gegebenenfalls substituiertem Alkyl sind gegebenenfalls verzweigte $C_1$- bis $C_6$-Alkylreste zu verstehen, die Hydroxy-, Halogen-, $C_1$- bis $C_4$-Alkoxy-, $C_1$- bis $C_4$-Alkylthio-, $C_1$- bis $C_3$-Alkanoyloxy-, $C_1$- bis $C_4$-Dialkylamino- oder $C_1$- bis $C_4$-Dialkylphosphano-Gruppen tragen können. Beispiele, die den Umfang der Erfindung nicht einschränken sollen, sind : Methyl, Ethyl, 2-Propyl, Hydroxymethyl, 3-Hydroxybutyl, Chlormethyl, 3,5-Dichlor-2-hexyl, Methoxymethyl, Butoxyethyl, Methylthiomethyl, Acetoxymethyl, Dimethylaminomethyl, 3-(Dimethylamino)-2-methyl-1-propyl, Diethylphosphanomethyl.

Unter gegebenenfalls substituiertem Cycloalkyl sind solche mit 5 bis 7 Ringgliedern zu verstehen, die gegebenenfalls noch eine Methylgruppe tragen können, beispielsweise Cyclohexyl.

Unter gegebenenfalls substituiertem Aralkyl sind solche mit einem $C_1$- bis $C_4$-Alkyl-Teil und einem gegebenenfalls durch Methyl, Methoxy oder Chlor substituierten Phenyl- oder Naphthyl-Rest zu verstehen, beispielsweise Benzyl, Phenethyl, 1-Naphthylmethyl.

Unter gegebenenfalls substituiertem Hetaralkyl sind solche mit einem $C_1$- bis $C_4$-Alkyl-Teil und einem gegebenenfalls durch Methyl, Methoxy oder Chlor substituierten, ungesättigten oder teilweise oder vollständig gesättigten Hetaryl-Rest der Furan-, Thiophen- oder Pyridin-Reihe zu verstehen. Beispiele, die den Umfang der Erfindung nicht einschränken sollen, sind : 2-Tetrahydrofurylmethyl, 2-Picolyl, 2-(4-Pyridyl)-ethyl.

Unter gegebenenfalls substituiertem Aryl sind solche der Naphthyl- und vorzugsweise Phenyl-Reihe zu verstehen, die $C_1$- bis $C_4$-Alkyl, $C_1$-bis $C_4$-Alkoxy, Chlor, Cyclohexyl, Phenyl, Phenoxy, Benzyloxy oder Hetaryl, dessen Bedeutung anschließend erläutert wird, tragen können, beispielsweise Phenyl, 3-Tolyl, Anisyl, 4-Cyclohexyl, Biphenylyl, 4-Benzoxazolylphenyl, 4-(2H-Benz[d][1,2,3]triazol-2-yl)-phenyl.

Unter gegebenenfalls substituiertem Hetaryl und gegebenenfalls substituiertem teilweise oder vollständig hydriertem Hetary sind Vertreter der Furan-, Thiophen-, Oxdiazol-, Pyrazol-, Thiadiazol-, Benzoxazol-, Benzthiazol-, Benzimidazol-, Benztriazol-, Naphthoxazol- und Naphthotriazol-Reihen zu verstehen, die $C_1$- bis $C_4$-Alkyl, Phenyl oder weiteres Hetaryl tragen können. Beispiele, die den Umfang der Erfindung nicht einschränken sollen sind :

5-Phenylthien-2-yl, Benz-1,3-oxazol-2-yl, 4-Methyl-2H-benz[d][1,2,3]triazol-2-yl, 2H-Naphtho[1,2-d][1,2,3]triazol-2-yl, 5-(Benzoxazolyl)-fur-2-yl.

Unter gegebenenfalls substituiertem Alkenyl sind solche mit 2-6 C-Atomen, vorzugsweise solche der 1-Alken-1-yl-Reihe zu verstehen, die verzweigt sein können und gegebenenfalls Methoxy oder Chlor tragen, beispielsweise 1-Propen-1-yl.

Unter gegebenenfalls substituiertem Arylalkenyl oder Hetarylalkenyl sind solche zu verstehen, die am $C_2$- bis $C_4$-Alkenyl-Teil Aryl- oder Hetaryl-Reste obiger Beschreibung tragen, beispielsweise Styryl, 3-Phenyl-1-propen-1-yl, 2-(Thien-2-yl)-ethen-1-yl, 2-(4-Benzoxazolylphenyl)-ethen-1-yl.

Unter Perhalogenalkyl sind solche mit 1-4, vorzugsweise 1-2 C-Atomen zu verstehen, die nur eine Art Halogen-Atome, vorzugsweise Fluor oder Chlor tragen, beispielsweise Trifluormethyl.

Unter gegebenenfalls substituiertem Alkoxy oder Alkylthio sind solche mit gegebenenfalls verzweigtem $C_1$- bis $C_4$-Alkyl-Teil zu verstehen, die gegebenenfalls weitere Alkoxy-Gruppen oder Phenyl tragen, beispielsweise Methoxy, 2-Propoxy, Benzyloxy, Methylthio.

Unter Halogen ist Fluor, Chlor oder Brom, vorzugsweise Chlor zu verstehen.

Unter einem gegebenenfalls substituierten Amino-Rest ist ein solcher mit 2 $C_1$- bis $C_4$-Alkyl- oder $C_1$- bis $C_2$-Aralkyl-Gruppen, die untereinander nicht gleich sein müssen, zu verstehen, beispielsweise Dimethylamino, Benzylethylamino.

Unter gegebenenfalls substituiertem Alkoxycarbonyl sind solche mit 1 bis 6 C-Atomen zu verstehen, die noch weitere $C_1$- bis $C_2$-Alkoxy-Gruppen oder Phenyl tragen können, beispielsweise Methoxycarbonyl, Methoxyethoxyethoxycarbonyl, Benzyloxycarbonyl.

Unter gegebenenfalls substituiertem Aryloxycarbonyl sind vorzugsweise solche der Phenyl-Reihe zu verstehen, die durch $C_1$- bis $C_3$-Alkyl, $C_1$- bis $C_3$-Alkoxy oder Chlor substituiert sein können, beispielsweise Phenoxycarbonyl.

Unter gegebenenfalls substituiertem Carbamyl ist ein solcher mit 2 $C_1$- bis $C_4$-Alkyl oder einer Tetra- oder Pentamethylen-Gruppe zu verstehen, wobei letztere zusammen mit dem Stickstoff einen Pyrrolidin- bzw. Piperidin-Ring bildet, beispielsweise Dimethylaminocarbonyl, Piperidinocarbonyl.

Unter gegebenenfalls substituiertem Alkylsulfonyl sind solche mit $C_1$- bis $C_6$-Alkyl zu verstehen, die durch Phenyl oder Halogen substituiert sein können, beispielsweise Methylsulfonyl, Benzylsulfonyl, Trifluormethylsulfonyl.

Unter gegebenenfalls substituiertem Aryl- oder Hetarylsulfonyl sind solche zu verstehen, deren Aryl- oder Hetaryl-Reste den oben angegebenen Reihen entstammen. Beispiele, die den Umfang der Erfindung nicht einschränken sollen, sind : Phenylsulfonyl, 4-Chlor-phenylsulfonyl.

Unter gegebenenfalls substituiertem Aminosulfonyl sind solche mit 2 $C_1$- bis $C_6$-Alkyl-Resten, die nicht gleich zu sein brauchen, zu verstehen, beispielsweise Dimethylaminosulfonyl.

Unter gegebenenfalls substituiertem Acyl-Rest sind $C_1$- bis $C_4$-Alkanoyl- oder $C_3$- bis $C_4$-Alkenoyl-Reste zu verstehen, die Methoxy, Chlor oder Phenyl tragen können, beispielsweise Acetyl, Phenylethanoyl, 3-Phenyl-2-proprenoyl, benzoyl.

Unter Dialkoxyphosphoryl sind solche mit 2 $C_1$- bis $C_4$-Alkoxy-Resten zu verstehen die nicht gleich zu sein brauchen, beispielsweise Dimethoxyphosphoryl.

Als an den Triazol-Ring anellierte Ringe kommen in Frage $C_5$- bis $C_7$-Cycloalkene, $C_5$- bis $C_7$-Cycloalkenone, $C_5$- bis $C_7$-Benzocycloalkadiene, Benzol, Naphthalin, 1,2-Diazol, 2H-Benzo[d][1,2,3]triazol, Uracil, Benzo[b]pyran-2-on, 1,2-Dihydrobenzo[b]pyrid-2-on, Benzofuran, Benzothiophen und Benzothiophendioxid, die durch $C_1$- bis $C_{12}$-Alkyl, $C_1$- bis $C_{12}$-Alkoxy, Halogen und/oder Phenyl substituiert sein können.

Beispiele sind :

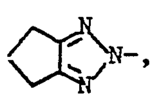, 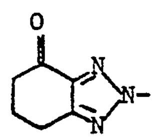, 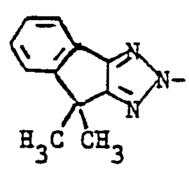, 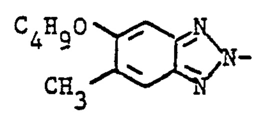,

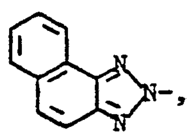, 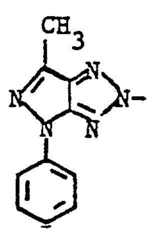, 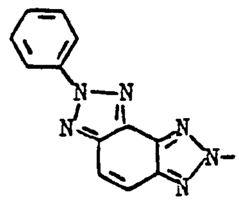, 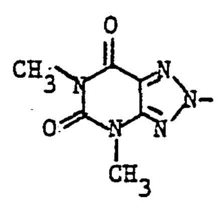,

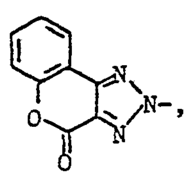, 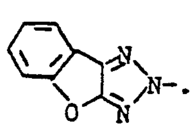.

Besonders bevorzugte Verfahrensprodukte sind 2-(2,2,2-Trichlor-1-hydroxy-ethyl)-phenole der Formel

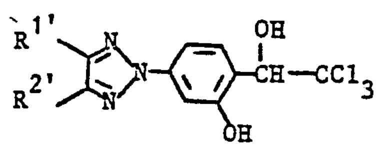 (III)

worin

$R^{1'}$ Wasserstoff, $C_1$- bis $C_6$-Alkyl, gegebenenfalls durch $C_1$- bis $C_4$-Alkyl oder $C_1$- bis $C_4$-Alkoxy oder Chlor substituiertes Phenyl oder Chlor und

$R^{2'}$ $C_1$- bis $C_6$-Alkyl, $C_1$- bis $C_6$-Alkoxy, gegebenenfalls durch $C_1$- bis $C_4$-Alkyl oder $C_1$- bis $C_4$-Alkoxy oder Chlor substituiertes Phenyl, Biphenylyl, Cyano, Alkoxycarbonyl, wobei die Alkoxygruppe 1 bis 12 C-Atome enthält oder durch 2 $C_1$- bis $C_6$-Alkylgruppen substituiertes Carbamoyl oder

$R^{1'}$ und $R^{2'}$ gemeinsam die noch fehlenden Glieder eines gegebenenfalls durch $C_1$- bis $C_{12}$-Alkyl und/oder $C_1$- bis $C_{12}$-Alkoxy substituierten anellierten Benzo- oder Naphtho-Ringes bedeuten.

Von ganz besonderem Interesse sind folgende Beispiele :

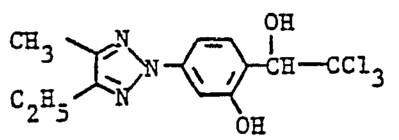 ,

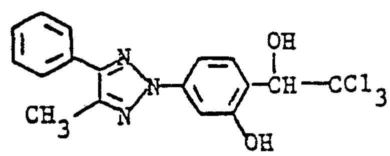 ,

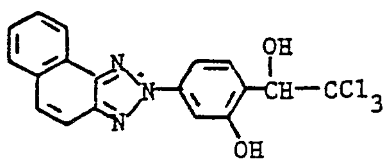

Die Phenole der Formel IV sind entweder literaturbekannt (Liebig Ann. Chem. *1978,* 345) oder lassen sich auf bekannte Weise, z. B. durch Diazotierung entsprechender Anilin-Derivate und Verkochen der dabei entstehenden Diazoniumsalze, herstellen.

Die erfindungsgemäßen 2-(2,2,2-Trichlor-1-hydroxy-ethyl)-phenole der Formel I stellen wertvolle Ausgangsprodukte zur Darstellung von Salicylaldehyden der formel

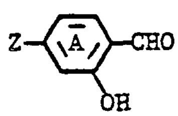 (VII)

dar, worin

Z und A die oben angegebene Bedeutung haben.

Dieses Verfahren ist dadurch gekennzeichnet, daß man auf 2-(2,2,2-Trichlor-1-hydroxy-ethyl)-phenole der Formel I in an sich bekannter Weise chloroformabspaltende Reagenzien einwirken läßt (vgl. Houben-Weyl, Methoden der organischen Chemie, Band VII/1, S. 329 ff.).

Die aus 2-(2,2,2-Trichlor-1-hydroxy-ethyl)-phenolen der Formel I durch Chloroform-Abspaltung erhältlichen Salicylaldehyde der Formel VII sind praktisch durchweg literaturbekannt (z. B. DE-OS 23 35 218, DE-OS 28 48 670) und eignen sich vor allem als Ausgangsmaterialien zur Herstellung von optischen Aufhellern, Fluoreszenzfarbstoffen, Laserfarbstoffen und Lichtsammlern für Displays, wie sie z. B. in DE-AS 10 20 636, DE-AS 12 82 591, DE-OS 14 70 242, DE-OS 15 19 471, DE-OS 15 94 845, DE-OS 15 94 851, DE-OS 16 70 975, DE-OS 16 70 999, DE-OS 16 95 817, DE-OS 19 32 256, DE-OS 20 29 157, DE-OS 20 37 854, DE-OS 20 47 547, DE-OS 21 61 343, DE-OS 28 16 028, DE-OS 28 21 116, DE-OS 28 48 670 beschrieben sind.

## Beispiel 1

251,3 g 2-(3-Hydroxyphenyl)-4-methyl-5-phenyl-2H-1,2,3-triazol werden bei 90 °C in 800 ml Toluol weitgehend gelöst und 2,0 g Natriumhydroxid-Pulver zugesetzt. Durch Refluxieren am Wasserabscheider werden 0,8 g Wasser ausgekreist. Nach Abkühlen auf 90 °C setzt man 6,7 g wasserfreies Aluminiumtrichlorid zu, erhitzt für 15 min zum Rückfluß und kühlt auf Raumtemperatur ab. Während 45 min tropft man bei ca. 20 °C 147,4 g Trichloracetaldehyd zu und rührt 15 h bei Raumtemperatur. Die dicke Suspension wird auf 0 °C gekühlt, abgesaugt und mit 25 ml Toluol gewaschen. Das hellgraue Kristallisat wird bei 60 °C im Vakuum getrocknet Ausbeute : 399 g = 100 % der Theorie 2-(3-Hydroxy-4-(2,2,2-trichlor-1-hydroxy-ethyl)-phenyl)-4-methyl-5-phenyl-2H-1,2,3-triazol vom Schmelzpunkt 181-182 °C.

Ber. : C 51,2 ; H 3,5 ; N 10,5 ; Cl 26,7

Gef. : C 50,5 ; H 3,4 ; N 10,2 ; Cl 27,0

Das Produkt enthält laut Analyse noch 2,2 % Salze (Natrium- und Aluminiumsalze), ist also für weitere Umsetzungen direkt verwendbar.

## Beispiel 2

251,3 g 2-(3-Hydroxyphenyl)-4-methyl-5-phenyl-2H-1,2,3-triazol werden bei 90 °C in 800 ml Toluol weitgehend gelöst und 2,8 g Kaliumhydroxid-Pulver zugesetzt. Durch Refluxieren am Wasserabscheider werden 0,9 g Wasser ausgekreist. Nach Abkühlen auf 90 °C setzt man 6,7 g wasserfreies Aluminiumtrichlorid zu, erhitzt für 15 min zum Rückfluß und kühlt auf 35-40 °C ab. Während 45 min tropft man bei 35-40 °C 147,4 g Trichloracetaldehyd zu und rührt 2 h bei 40 °C. Die dicke Suspension wird auf 0 °C gekühlt, abgesaugt und mit 25 ml Toluol gewaschen. Das hellgraue Kristallisat wird bei 60 °C im Vakuum getrocknet.

Ausbeute : 395 g = 98,9 % der Theorie 2-(3-Hydroxy-4-(2,2,2-trichlor-1-hydroxy-ethyl)-4-methyl-5-phenyl-2H-1,2,3-triazol vom Schmelzpunkt 181-182 °C.

Umkristallisation aus Toluol unter Zusatz von Bleicherde liefert 352 g (88 % der Theorie) schwach rosagefärbtes Kristallisat vom Schmelzpunkt 185-186 °C.

Auf analoge Weise lassen sich die Verbindungen der Beispiele 3-7 herstellen :

| Beispiel | Verbindung | Ausbeute |
|---|---|---|
| 3 | 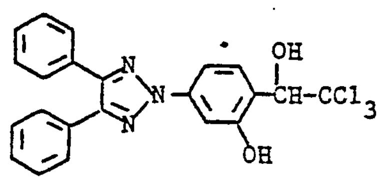 | 95 % |
| 4 | 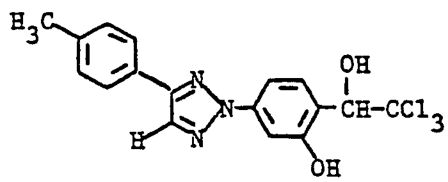 | 85 % |
| 5 | 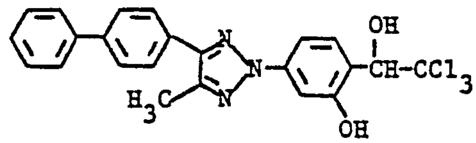 | 91 % |
| 6 | 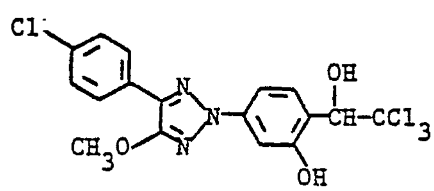 | 87 % |
| 7 | 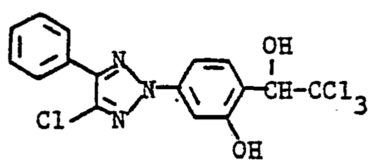 | 81 % |

## Beispiel 8

20,3 g 2-(3-Hydroxyphenyl)-4-ethyl-5-methyl-2H-1,2,3-triazol werden bei 90 °C in 60 ml Toluol gelöst und 0,28 g Kaliumhydroxid-Pulver zugesetzt. Durch Refluxieren am Wasserabscheider werden 0,09 g Wasser ausgekreist. Nach Abkühlen auf 90 °C setzt man 0,67 g wasserfreies Aluminiumtrichlorid zu, erhitzt für 15 min zum Rückfluß und kühlt auf 35-40 °C ab. Während 45 min tropft man bei 35-40 °C 14,8 g

Trichloracetaldehyd zu und rührt 2 h bei 40 °C. Die dicke Suspension wird auf 0 °C gekühlt, abgesaugt und mit 3 ml Toluol gewaschen. Das farblose Kristallisat wird bei 60 °C im Vakuum getrocknet.

Ausbeute : 34,4 g = 98,2 % der Theorie 2-(3-Hydroxy-4-(2,2,2-trichlor-1-hydroxy-ethyl)-phenyl)-4-ethyl-5-methyl-2H-1,2,3-triazol vom Schmelzpunkt 158-163 °C.

Umkristallisation aus Toluol unter Zusatz von Bleicherde liefert 18,0 g (80 %) schwach rosagefärbtes Kristallisat vom Schmelzpunkt 166-168 °C. IR (KBr) : 3 320, 2 925, 2 855, 1 639, 1 615, 1 524 $cm^{-1}$. $^1$H-NMR ($[D_6]$ DMSO) : $\delta$ = 1,27 (t, J = 7,5 Hz ; 3H, $CH_3CH_2$—), 2,30 (s ; 3H, $CH_3$), 2,68 (q, J = 7,5 Hz ; 2H, $CH_3CH_2$—), 5,70 (d, J = 6 Hz ; 1H, CHOH), 7,15 (d, J = 6 Hz ; 1H, CHOH), 7,25-7,9 (m ; 3H, Aromaten), 10,34 (s ; 1H, OH).

Ms : m/e = 349 (1 %, $M^+$), 232 (100 %,$M^+$-$CCl_3$).

Ber. : C 44,5 ; H 4,0 ; N 12,0 ; Cl 30,3

Gef. : C 44,9 ; H 4,0 ; N 12,2 ; Cl 30,3.

Auf analogem Weg lassen sich die Verbindungen der Beispiele 9-12 herstellen :

| Beispiel | Verbindung | Ausbeute |
|---|---|---|
| 9 | 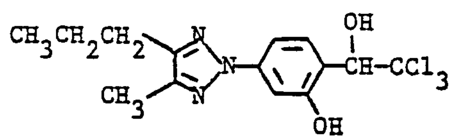 | 95 % |
| 10 | 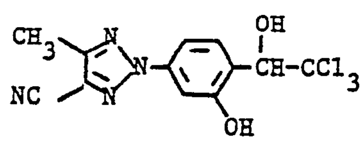 | 91 % |
| 11 | 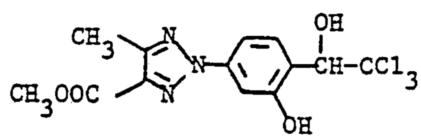 | 92 % |
| 12 | 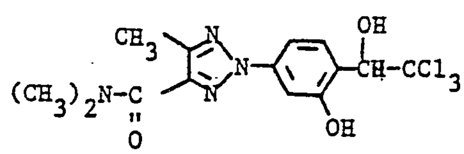 | 95 % |

Beispiel 13

39,8 g 2-(3-Hydroxy-4-(2,2,2-trichlor-1-hydroxy-ethyl)-phenyl)-4-methyl-5-phenyl-2H-1,2,3-triazol werden in 20 ml Methanol mit 18 g 30 %iger Natriummethylatlösung versetzt. Nach 30 min Rühren wird das Solvens i. Vak. bei Raumtemperatur abgezogen und das farblose Kristallisat in 100 ml wasserfreiem Dimethylformamid aufgenommen. Bei 30 °C tropft man 7,2 g 30 %ige Natriummethylatlösung zu und gießt die gelbe Lösung nach 17 h auf 1 l Eiswasser, stellt mit Essigsäure auf pH 5-6, saugt das beige Kristallisat ab und trocknet im Vakuum bei 100 °C. Ausbeute : 25,9 g = 93 % der Theorie 2-Hydroxy-4-(4-methyl-5-phenyl-2H-1,2,3-triazol-2-yl)-benzaldehyd vom Schmelzpunkt 120-121 °C.

Umkristallisation aus Toluol liefert 21 g = 75,5 % der Theorie vom Schmelzpunkt 123-124 °C.

Literatur-Schmelzpunkt : 121-123 °C (DE-OS 28 48 670).

Beispiel 14

39,8 g 2-(3-Hydroxy-4-(2,2,2-trichlor-1-hydroxy-ethyl)-phenyl)-4-methyl-5-phenyl-2H-1,2,3-triazol werden in 55 ml wasserfreiem DMF gelöst und bei 30 °C während 1,5 h portionsweise mit 5,4 g Natriumhydroxid-Pulver versetzt. Die dicke gelbe Suspension wird 1 h bei 30 °C gerührt, auf 0 °C gekühlt, abgesaugt und mit 5 ml DMF und 100 ml Wasser gewaschen. Nach Trocknen bei 100 °C erhält man 23,3 g 2-Hydroxy-4-(4-methyl-5-phenyl-2H-1,2,3-triazol-2-yl)-benzaldehyd-Na-Salz mit einem Reingehalt von 93 %, also in 72 % Ausbeute, vom Schmelzpunkt 286-289 °C. Das Produkt wird in Wasser suspendiert und mit Essigsäure auf pH 5-6 gebracht. Absaugen und Trocknen bei 100 °C i. Vak. liefert so 21,6 g 2-Hydroxy-4-(4-methyl-5-phenyl-2H-1,2,3-triazol-2-yl)-benzaldehyd vom Schmelzpunkt 123-124 °C. (aus Toluol)

Beispiel 15

39,8 g 2-(3-Hydroxy-4-(2,2,2-trichlor-1-hydroxy-ethyl)-phenyl)-4-methyl-5-phenyl-2H-1,2,3-triazol werden in 60 ml wasserfreiem Dimethylformamid gelöst. Hierzu tropft man binnen 1 h 25,2 g 30 %ige Natriummethylatlösung bei 25 °C ein und destilliert gleichzeitig aus der Reaktionsmischung bei 15 Torr Methanol und Chloroform ab. Nach insgesamt 2 h wird der dicke gelbe Brei auf 0 °C gekühlt, abgesaugt und mit 5 ml DMF und 100 ml Wasser gewaschen. Nach Trocknen bei 100 °C i. Vak. erhält man 26,1 g 2-Hydroxy-4-(4-methyl-5-phenyl-2H-1,2,3-triazol-2-yl)-benzaldehyd-Na-Salz in 91 %iger Reinheit, also in 79 % Ausbeute, vom Schmelzpunkt 285-289 °C (Zers.).

Die Überführung in die OH-Form kann wie in Beispiel 14 beschrieben erfolgen.

Beispiel 16

39,8 g 2-(3-Hydroxy-4-(2,2,2-trichlor-1-hydroxy-ethyl)-phenyl)-4-methyl-5-phenyl-2H-1,2,3-triazol werden in 50 ml wasserfreiem DMF gelöst und bei 25-30 °C während 2,5 h portionsweise mit 8,1 g Natriummethylat-Pulver versetzt. Der dicke gelbe Brei wird 15 min auf 60 °C erwärmt, auf 0 °C abgekühlt und abgesaugt. Nach Waschen mit 5 ml DMF, 100 ml Wasser und Trocknen bei 100 °C i.Vak. erhält man 25,4 g 2-Hydroxy-4-(4-Methyl-5-phenyl-2H-1,2,3-triazol-2-yl)-benzaldehyd-Na-Salz in 93 %iger Reinheit, also in 78 % Ausbeute, vom Schmelzpunkt 287-289 °C (Zers.).

Die Überführung in die OH-Form kann wie in Beispiel 14 beschrieben erfolgen.

Analog lassen sich die Verbindungen der Beispiele 17-21 herstellen :

| Beispiel | Verbindung |
| --- | --- |
| 17 | 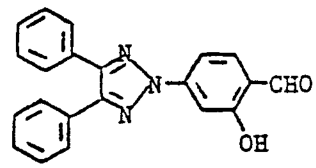 |
| 18 | 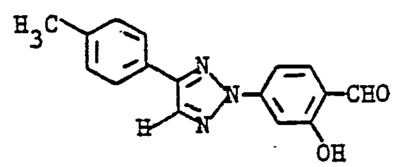 |
| 19 | 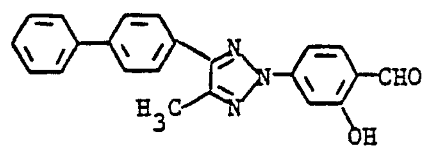 |
| 20 | 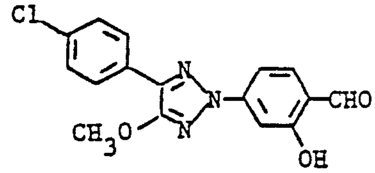 |
| 21 | 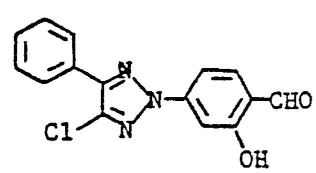 |

Beispiel 22

39,8 g 2-(3-Hydroxy-4-(2,2,2-trichlor-1-hydroxy-ethyl)-phenyl)-4-methyl-5-phenyl-2H-1,2,3-triazol werden in 80 ml Tetramethylharnstoff gelöst und bei 25-30 °C während 2,5 h portionsweise mit 8,1 g Natriummethylat-Pulver versetzt. Der dicke gelbe Brei wird auf 0 °C gekühlt, abgesaugt und mit 5 ml Tetramethylharnstoff und 100 ml Wasser gewaschen. Nach Trocknen bei 100 °C i. Vak. erhält man 23,5 g 2-Hydroxy-4-(4-methyl-5-phenyl-2H-1,2,3-triazol-2-yl)-benzaldehyd-Na-Salz in 92 %iger Reinheit, also in 72 % Ausbeute, vom Schmelzpunkt 286-289 °C (Zers.).

Beispiel 23

35,0 g 2-(3-Hydroxy-4-(2,2,2-trichlor-1-hydroxy-ethyl)-phenyl)-4-ethyl-5-methyl-2H-1,2,3-triazol werden in 50 ml wasserfreiem DMF gelöst und bei 25-30 °C während 2,5 h portionsweise mit 8,1 g Natriummethylat-Pulver versetzt. Der dicke gelbe Brei wird 10 min auf 60 °C erwärmt, nach Abkühlen in 200 ml Eiswasser gegossen und mit Essigsäure auf pH 5-6 gebracht. Absaugen und Trocknen bei 100 °C i. Vak. liefert 18,0 g = 71 % der Theorie blaß gelben 2-Hydroxy-4-(4-ethyl-5-methyl-2H-1,2,3-triazol-2-yl)-benzaldehyd vom Schmelzpunkt 85-87 °C.

Umkristallisation aus Methylcyclohexan oder Toluol ergibt 14,9 g (59 %) vom Schmelzpunkt 91-92 °C. Lit.-Schmp. : 86-88 °C (DE-OS 28 48 670).

Analog lassen sich die Verbindungen der Beispiele 24-27 herstellen :

| Beispiel | Verbindung |
|---|---|
| 24 | 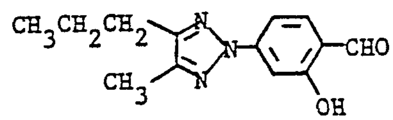 |
| 25 | 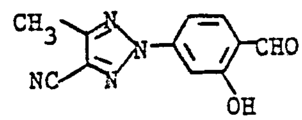 |
| 26 | 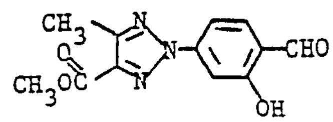 |
| 27 | 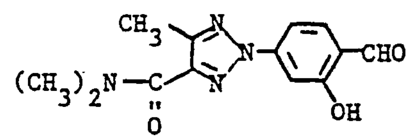 |

Beispiel 28

40,9 g 2-(3-Hydroxy-4-(2,2,2-trichlor-1-hydroxy-ethyl)-2H-naphtho[1,2-d]-[1,2,3]triazol werden in 200 ml wasserfreiem Dimethylformamid gelöst. Hierzu tropft man während 1 h 25,2 g 30 %ige Natriummethylatlösung bei 25 °C und destilliert gleichzeitig aus der Reaktionsmischung bei 15 Torr Methanol und Chloroform ab. Nach insgesamt 2 h wird der dicke orangegelbe Brei auf 0 °C gekühlt, abgesaugt und mit 5 ml DMF und 200 ml Wasser gewaschen. Nach Trocknen bei 100 °C erhält man 19,0 g 2-Hydroxy-4-(2H-naphtho[1,2-d][1,2,3]triazol-2-yl)-benzaldehyd-Na-Salz in 93 %iger Reinheit, also in 57 % Ausbeute, vom Schmelzpunkt 290-293 °C (Zers.). Das Produkt wird in Wasser suspendiert und mit Essigsäure auf pH 5-6 gebracht. Absaugen und Trocknen bei 100 °C liefert so 17,7 g (57 % d. Th.) 2-Hydroxy-4-(2H-naphtho[1,2-d][1,2,3]triazol-2-yl)-benzaldehyd als gelbes Pulver vom Schmelzpunkt 255 °C. Umkristallisation aus Chlorbenzol ergibt 11,4 g (40 %) vom Schmelzpunkt 256-257 °C.

Beispiel 29

Analog erhält man 2-Hydroxy-4-(5-(1-butoxy)-6-methyl-2H-benzo[d][1,2,3]-triazol-2-yl)-benzaldehyd in 63 % Ausbeute.

Beispiel 30

251,3 g 2-(3-Hydroxyphenyl)-4-methyl-5-phenyl-2H-1,2,3-triazol werden bei 90 °C in 800 ml Toluol weitgehend gelöst und 2,8 g Kaliumhydroxid-Pulver zugesetzt. Durch Refluxieren am Wasserabscheider werden 0,9 g Wasser ausgekreist. Nach Abkühlen auf 90 °C setzt man 6,7 g wasserfreies Aluminiumtrichlorid zu, erhitzt für 15 min zum Rückfluß und kühlt auf 35-40 °C ab. Während 45 min tropft man bei 35-40 °C 147,4 g Trichloracetaldehyd zu und rührt 2 h bei 40 °C. Die dicke Suspension wird auf 0 °C gekühlt,

abgesaugt und mit 25 ml Toluol gewaschen. Das weitgehend trocken gesaugte Kristallisat (Toluolgehalt ca. 250 g) wird in 500 ml wasserfreiem Dimethylformamid gelöst und bei 25-30 °C während 2,5 h portionsweise mit 81 g Natriummethylat-Pulver versetzt. Der dicke gelbe Brei wird 15 min auf 60 °C erwärmt, auf 0 °C abgekühlt und abgesaugt. Nach Waschen mit 50 ml Dimethylformamid, 1 000 ml Wasser und Trocknen bei 100 °C i. Vak. erhält man 262 g 2-Hydroxy-4-(4-methyl-5-phenyl-2H-1,2,3-triazol-2-yl)-benzaldehyd-Na-Salz in 93 %iger Reinheit, also in 81 % Ausbeute, vom Schmelzpunkt 286-289 °C (Zers.).

Die Überführung in die OH-Form kann wie in Beispiel 14 beschrieben erfolgen.

**Ansprüche**

1. Verfahren zur Herstellung von 2-(2,2,2-Trichlor-1-hydroxy-ethyl)-phenolen der Formel

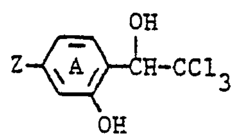

worin

Z einen Rest der 1,2-Diazol-1-yl-, 1,2,3-Triazol-1-yl-, 1,2,3-Triazol-2-yl-, 1,2,4-Triazol-1-yl- und 1,2,4-Triazol-4-yl-Reihe und

A einen Benzolring, der gegebenenfalls durch $C_1$- bis $C_2$-alkyl, $C_1$- bis $C_2$-Alkoxy, Halogen oder einen anellierten Benzolring substituiert sein kann, bedeuten,
dadurch gekennzeichnet, daß man Phenole der Formel

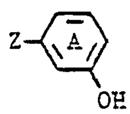

worin

Z und A die oben angegebene Bedeutung besitzen, in Gegenwart von 0,02-0,1 Moläquivalenten ihrer Alkali- oder Erdalkalisalze und Lewis-Säuren mit Trichloracetaldehyd umsetzt.

2. Verfahren nach Anspruch 1, dadurch gekennzeichnet, daß man 2-(2,2,2-Trichlor-1-hydroxy-ethyl)-phenole der Formel

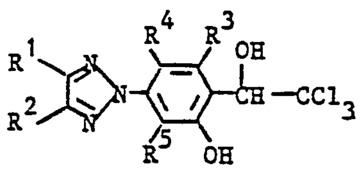

worin

$R^1$ und $R^2$ unabhängig voneinander Wasserstoff, einen gegebenenfalls substituierten Alkyl-, Cycloalkyl-, Aralkyl-, Hetaralkyl-, Aryl-, Hetaryl-, teilweise oder vollständig hydrierten Hetary-, Alkenyl-, Arylalkenyl- oder Hetarylalkenylrest, Perhalogenalkyl, Hydroxy, einen gegebenenfalls substituierten Alkoxy- oder Alkylthio-Rest, Halogen, einen gegebenenfalls substituierten Amino-Rest, Cyano, einen gegebenenfalls substituierten Alkoxycarbonyl-, Aryloxy- carbonyl-, Carbamyl-, Alkylsulfonyl-, Arylsulfonyl-, Hetarylsulfonyl-, Aminosulfonyl- oder Acyl-Rest, Di-alkoxyphosphoryl oder gemeinsam die noch fehlenden Glieder eines gegebenenfalls substituierten oder Keto-Gruppen und/oder ein oder mehrere Heteroatome enthaltenden gegebenenfalls durch einen gegebenenfalls substituierten Benzol-Ring oder Heterocyclus anellierten ungesättigten oder teilweise oder vollständig gesättigten Ring und

$R^3$, $R^4$ und $R^5$ unabhängig voneinander Wasserstoff, Methyl, Methoxy oder Chlor bedeuten, herstellt.

3. Verfahren nach Anspruch 1, dadurch gekennzeichnet, daß man 2-(2,2,2-Trichlor-1-hydroxy-ethyl)-phenole der Formel

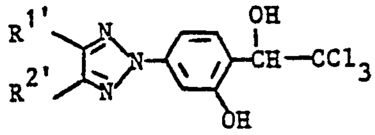

worin

$R^{1'}$ Wasserstoff, $C_1$- bis $C_6$-Alkyl, gegebenenfalls durch $C_1$- bis $C_4$-Alkyl oder $C_1$- bis $C_4$-Alkoxy oder Chlor substituiertes Phenyl oder Chlor und

$R^{2'}$ $C_1$- bis $C_6$-Alkyl, $C_1$- bis $C_6$-Alkoxy, gegebenenfalls durch $C_1$- bis $C_4$-Alkyl oder $C_1$- bis $C_4$-Alkoxy oder Chlor substituiertes Phenyl, Biphenylyl, Cyano, Alkoxycarbonyl, wobei die Alkoxy-Gruppe 1 bis 12 C-Atome enthält oder durch 2 $C_1$- bis $C_6$-Alkyl-Gruppen substituiertes Carbamyl oder

$R^{1'}$ und $R^{2'}$ gemeinsam die noch fehlenden Glieder eines gegebenenfalls durch $C_1$- bis $C_{12}$-Alkyl und/oder $C_1$- bis $C_{12}$-Alkoxy substituierten anellierten Benzo- oder Naphtho-Rings bedeuten, herstellt.

**Claims**

1. Process for the preparation of 2-(2,2,2-trichloro-1-hydroxy-ethyl)-phenols of the formula

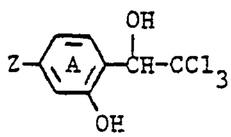

wherein

Z denotes a radical of the 1,2-diazol-1-yl, 1,2,3-triazol-1-yl, 1,2,3-triazol-2-yl, 1,2,4-triazol-1-yl and 1,2,4-triazol-4-yl series and

A denotes a benzene ring which can optionally be substituted by $C_1$- to $C_2$-alkyl, $C_1$- to $C_2$-alkoxy, halogen or a fused-on benzene ring,

characterised in that phenols of the formula

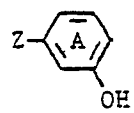

wherein

Z and A have the abovementioned meaning, are reacted with trichloroacetaldehyde in the presence of 0.02-0.1 molar equivalents of their alkali metal or alkaline earth metal salts and Lewis acids.

2. Process according to Claim 1, characterised in that 2-(2,2,2-trichloro-1-hydroxy-ethyl)-phenols of the formula

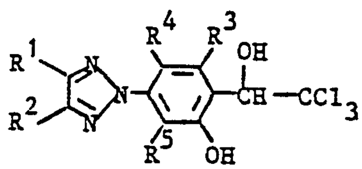

wherein

$R^1$ and $R^2$ independently of one another denote hydrogen, an optionally substituted alkyl, cycloalkyl, aralkyl, hetaralkyl, aryl, hetaryl, partially or completely hydrogenated hetaryl, alkenyl, arylalkenyl or hetaryl-alkenyl radical, perhalogenoalkyl, hydroxyl, an optionally substituted alkoxy or alkylthio radical, halogen, an optionally substituted amino radical, cyano, an optionally substituted alkoxycarbonyl, aryloxycarbonyl, carbamyl, alkylsulphonyl, arylsulphonyl, hetarylsulphonyl, amino-sulphonyl or acyl radical, or dialkoxyphosphoryl, or together denote the still missing members of an unsaturated or partially or completely satured ring which is optionally substituted or contains keto groups and/or one or more hetero-atoms and is optionally fused to an optionally substituted benzene ring or heterocyclic ring and

$R^3$, $R^4$ and $R^5$ independently of one another denote hydrogen, methyl, methoxy or chlorine, are prepared.

3. Process according to Claim 1, characterised in that 2-(2,2,2-trichloro-1-hydroxy-ethyl)-phenols of the formula

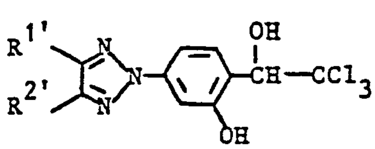

wherein

$R^{1'}$ denotes hydrogen, $C_1$- to $C_6$-alkyl, phenyl which is optionally substituted by $C_1$- to $C_4$-alkyl or $C_1$- to $C_4$-alkoxy or chlorine, or chlorine and

$R^{2'}$ denotes $C_1$- to $C_6$-alkyl, $C_1$- to $C_6$-alkoxy, phenyl which is optionally substituted by $C_1$- to $C_4$-alkyl or $C_1$- to $C_4$-alkoxy or chlorine, biphenylyl, cyano, alkoxycarbonyl, the alkoxy group containing 1 to 12 C atoms, or carbamyl which is substituted by 2 $C_1$- to $C_6$-alkyl groups,

or

$R^{1'}$ and $R^{2'}$ together denote the still missing members of a fused-on benzo or naphtho ring which is optionally substituted by $C_1$- to $C_{12}$-alkyl and/or $C_1$- to $C_{12}$-alkoxy, are prepared.

**Revendications**

1. Procédé de production de 2-(2,2,2-trichloro-1-hydroxyéthyl)-phénols de formule

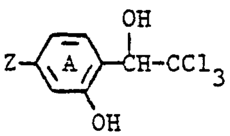

dans laquelle

Z est un reste de la série 1,2-diazole-1-yle, 1,2,3-triazole-1-yle, 1,2,3-triazole-2-yle, 1,2,4-triazole-1-yle et 1,2,4-triazole-4-yle et

A est un noyau benzénique qui peut être substitué le cas échéant par un groupe alkyle en $C_1$ ou $C_2$, alkoxy en $C_1$ ou $C_2$, un halogène ou un noyau benzénique condensé,

caractérisé en ce qu'on fait réagir des phénols de formule

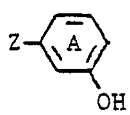

dans laquelle

Z et A ont la définition donnée ci-dessus, en présence de 0,02 à 0,1 équivalent molaire de leurs sels alcalins ou alcalino-terreux et d'acides de Lewis avec le trichloracétaldéhyde.

2. Procédé suivant la revendication 1, caractérisé en ce qu'on produit des 2-(2,2,2-trichloro-1-hydroxyéthyl)-phénols de formule

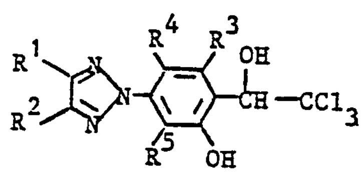

dans laquelle

$R^1$ et $R^2$ représentent, indépendamment l'un de l'autre, l'hydrogène, un reste alkyle, cycloalkyle, aralkyle, hétéralkyle, aryle, hétaryle, hétaryle partiellement ou totalement hydrogéné, alcényle, arylalcényle ou hétarylalcényle éventuellement substitué, un reste perhalogénalkyle, hydroxy, un reste alkoxy ou alkylthio éventuellement substitué, un halogène, un reste amino éventuellement substitué, un reste cyano, un reste alkoxycarbonyle, aryloxycarbonyle, carbamyle, alkylsulfonyle, arylsulfonyle, hétérylsulfonyle, aminosulfonyle ou acyle éventuellement substitué, un reste dialkoxy-phosphoryle, ou forment ensemble les chaînons encore manquants d'un noyau insaturé ou partiellement ou totalement saturé, éventuellement substitué ou portant des groupes céto et/ou un ou plusieurs hétéroatomes, éventuellement condensé avec un noyau benzénique ou un hétérocycle éventuellement substitué et

$R^3$, $R^4$ et $R^5$ représentent, indépendamment l'un de l'autre, l'hydrogène, un groupe méthyle, méthoxy ou du chlore.

3. Procédé suivant la revendication 1, caractérisé en ce qu'on produit des 2-(2,2,2-trichloro-1-hydroxyéthyl)-phénols de formule

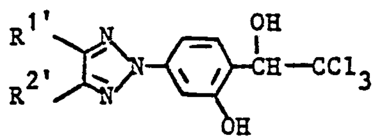

dans laquelle

$R^{1'}$ représente l'hydrogène, un groupe alkyle en $C_1$ à $C_6$, un groupe phényle portant éventuellement un substituant alkyle en $C_1$ à $C_4$ ou alkoxy en $C_1$ à $C_4$ ou chloro, ou du chlore et

$R^{2'}$ est un groupe alkyle en $C_1$ à $C_6$, alkoxy en $C_1$ à $C_6$, phényle éventuellement substitué par un radical alkyle en $C_1$ à $C_4$ ou alkoxy en $C_1$ à $C_4$ ou chloro, un groupe biphénylyle, cyano, alkoxycarbonyle, le groupe alkoxy comportant 1 à 12 atomes de carbone, ou un groupe carbamyle éventuellement substitué par deux groupes alkyle en $C_1$ à $C_5$, ou bien

$R^{1'}$ et $R^{2'}$ forment ensemble les chaînons encore manquants d'un noyau de benzène ou de naphtalène condensé, éventuellement substitué par un radical alkyle en $C_1$ à $C_{12}$ et/ou alkoxy en $C_1$ à $C_{12}$.